# EUROPEAN PATENT APPLICATION

(11) **EP 4 610 660 A1**
(43) Date of publication of application: **03.09.2025**
(21) Application number: 24160441.2
(22) Date of filing: 29.02.2024
(51) Int. Cl.: G01N 35/00, G01N 35/02, G01N 27/00, G01N 27/414, G01N 33/487

(54) **INDUCTION DRIVEN SENSOR, TEST APPARATUS AND A METHOD FOR SENSING SUBSTANCES WITHIN A SAMPLE**

(71) Applicant: Siemens Healthineers AG, 91301 Forchheim (DE)
(72) Inventor: Dethlefsen, Mark, 91058 Erlangen (DE); Seliger, Ramona, 91346 Wiesenttal-Muggendorf (DE)
(74) Representative: Siemens Healthineers Patent Attorneys

(57) **Abstract**

An induction driven sensor (1) substances within a sample to be analysed filled within a sample container (5), said induction driven sensor (1) comprising at least one low power consumption electrical sensor element (2) adapted to sense substances within a sample to be analysed; and an induction driven power supply structure (3) adapted to remotely power and read out the low power supply electrical sensor element (2) .

## Description

The invention related to an induction driven sensor, in particular for a test laboratory apparatus. The present invention is further related to a test apparatus and a method for sensing substances within a sample to be analysed.

Immunoassays (IA) are a common laboratory test and millions of tests are performed each day. A multitude of central lab, high-throughput instruments exist from multiple vendors. An immunoassay is a biochemical test that measures the presence or concentration of a macromolecule or a small molecule in a solution through the use of an antibody (usually) or an antigen (sometimes). The molecule detected by the immunoassay is often referred to as an "analyte" and is in many cases a protein, although it may be other kinds of molecules, of different sizes and types, as long as the proper antibodies that have the required properties for the assay are developed.

Most, if not all, of these conventional systems use a cuvette form-factor and rely on some form of optical read-out (e.g. chemiluminescence). In general, samples are immobilised on a solid phase or on beads to remove background material in an antibody-antigen type reaction. Subsequently, a second antibody, conjugated to an optically active compound, is bound to the target in a sandwich-assay type approach. Cuvettes can be designed to hold samples for a spectroscopic measurement, where a beam of light is passed through the sample within the cuvette to measure the absorbance, transmittance, fluorescence intensity, fluorescence polarization, or fluorescence lifetime of the sample.

However, the required excitation and detection systems are often complex and involve expensive components such as lenses, cameras / detectors and light sources. In addition, they require extensive servicing and a controlled (dark) environment to avoid interference of background light.

Use of electrical sensors in a laboratory test system involves less complex components. Electrical sensors are in general less expensive and more robust against environmental influences.

However electrical sensing technologies usually require direct contact of the sensing area with the analyte. This is due to the small sensing depth of surface sensors and the need to modify the surface with specific catcher molecules to make the sensor analyte specific. This makes it difficult to use such sensors in high throughput cuvette-based laboratory test systems due to the need for a direct electrical contact between the sensor and the external environment in order to supply power and read out data.

Against this background, an object underlying the present invention is to provide an electrical sensing opportunity for analytes within a sample which are suitable for a high throughput sample container based laboratory test system.

This problem is solved according to the invention by an induction driven sensor having the features of claim 1 and/or by a test apparatus having the features of claim 13 and/or by a method having the features of claim 15.

According thereto, the following is provided:
- An induction driven sensor comprising: at least one low power consumption electrical sensor element adapted to sense substances within a sample to be analysed provided in a sample container; and an induction driven power supply structure adapted to remotely power and read out the low power supply electrical sensor element.
- A test apparatus comprising multiple sample containers to receive samples to be analysed by said test apparatus, wherein each sample container comprises a sensor tag having an induction driven sensor according to the first aspect of the present invention.
- A method for sensing substances within a sample to be analysed comprising the steps of: delivering the sample to be analysed to a low power consumption electrical sensor element of an induction driven sensor adapted to sense substances within the delivered sample; and applying an excitation radio wave to an induction driven power supply structure of said induction driven sensor to remotely power and read out the low power supply electrical sensor element of said induction driven sensor.

The test apparatus comprises a holder adapted to hold multiple sample containers each adapted to receive samples to be analysed by said test apparatus, wherein each sample container comprises at least one sensor tag having an induction driven sensor including at least one low power consumption electrical sensor element adapted to sense substances within a sample to be analysed within a sample container and including an induction driven power supply structure adapted to remotely power and read out the low power supply electrical sensor element of the induction driven sensor.

The invention further provides a computer program product comprising instructions stored on a data carrier of the computer program product and adapted to perform the sensing method according to the third aspect of the present invention when executed on a controller of a test apparatus.

The invention provides according to a further aspect a sample container comprising an attached sensor tag with an induction driven sensor having at least one low power consumption electrical sensor element adapted to sense substances within a sample to be analysed filled into the sample container and having an induction driven power supply structure adapted to remotely power and read out the low power supply electrical sensor element of the induction driven sensor of the sensor tag attached to the sample container.

The invention provides according to a further aspect an attachable sensor tag with an induction driven sensor having at least one low power consumption electrical sensor element adapted to sense substances within a sample to be analysed filled into a sample container and having an induction driven power supply structure adapted to remotely power and read out the low power supply electrical sensor element of the induction driven sensor of the sensor tag.

The induction driven sensor, the attachable sensor tag and the sample container as well as the sensing method and the test apparatus according to the present invention can be used for all types of testing involving charged molecules, e.g. molecular, antigen-/antibody, proteins.

The induction driven sensor, the attachable sensor tag and the sample container as well as the sensing method and the test apparatus according to the present invention combine the advantages of microelectronic sensor technology and a fully automated, high-throughput cuvette based central laboratory system.

Such an approach has multiple advantages, for examples fewer wash steps are required causing a reduced reagent use. In addition, labelling the analyte with detection tags (e.g. fluorescent molecules), which is common in current lab analysers will not be required.

Moreover, electrical sensors such as the induction driven sensor according to the present invention are stable against vibrations, reducing the need for re-alignment and requiring less maintenance and servicing.

Advantageous configurations and developments emerge from the further dependent claims and from the description with reference to the figures of the drawings.

In a possible embodiment of the induction driven sensor, the induction driven power supply structure is patterned alongside the low power consumption electrical sensor element on a flexible sensor tag. The flexible tag can be easily attached to a sample container.

In a possible embodiment of the induction driven sensor, the flexible sensor tag is provided within a sample container. This brings the low power consumption electrical sensor element of the induction driven sensor into physical contact with the sample to be analysed filled in the sample container.

In a possible embodiment of the induction driven sensor, the induction driven power supply structure comprises an RFID structure. This provides the advantage of using a reliable and robust technology.

In a possible embodiment of the induction driven sensor, the low power consumption electrical sensor element is adapted to receive for its operation an inductive power supply from the induction driven power supply structure in response to external excitation radio waves. This allows to provide a remote and wireless electrical supply power to the sensor element.

In a possible embodiment of the induction driven sensor, the low power consumption electrical sensor element is adapted to generate during its operation a measurement signal in response to substances of the sample to be analysed.

In a possible embodiment of the induction driven sensor, the measurement signal generated by the low power consumption electrical sensor element during its operation is detected by a radio wave detector as changes in the excitation field.

This allows to get wireless a measurement signal for further processing.

In a possible embodiment of the induction driven sensor, the low power consumption electrical sensor element comprises a graphene based field effect transistor (GFET) sensor element. The GFET sensor element consumes little electrical power during its operation, i.e. less than 1 Milliwatt. One or more GFET sensor elements can be integrated into a circuit including the induction driven power supply structure. Several GFET sensor elements can be integrated in a common sensor element array requiring only a small area on a chip. The GFET sensor elements are easy to fabricate and have a small size.

In a possible embodiment of the induction driven sensor, the graphene based field effect transistor sensor element comprises a graphene layer provided on a dielectric top layer of a substrate. The graphene layer is highly sensitive to changes of electrical charge densities in its vicinity.

In a possible embodiment of the induction driven sensor, a sensitive recognition layer adapted to capture at least one target substance in the sample to be analysed is provided on the graphene layer of said graphene based field effect transistor sensor element. Different sensitive recognition layers can be placed on the graphene layer to adapt the graphene based field effect transistor sensor element for different test applications and test use cases.

In a possible embodiment of the induction driven sensor, the sensitive recognition layer comprises receptors adapted to capture at least one target substance in the sample to be analysed.

In a possible embodiment of the induction driven sensor, the sample to be analysed is delivered to the low power consumption electrical sensor element by a sample container, in particular a cuvette. This allows for a high throughput for analysing a plurality of samples.

In a possible embodiment of the test apparatus, the apparatus comprises a sample container rotation unit adapted to hold and rotate the sample containers such that the sample containers pass sequentially a radio wave excitation source of the test apparatus adapted to apply excitation radio waves to the sensor tags attached to the sample containers. This has the advantage of providing a wireless remote power supply to the sensors of the sensor tags.

In a possible embodiment of the test apparatus, the apparatus further comprises a radio wave detector adapted to detect changes in the excitation field in response to a measurement signal generated by the low power consumption electrical sensor element of the induction driven sensor of the sensor tag attached to the sample container passing concurrently the radio wave detector. This has the advantage of collecting wireless measurement signals from sensors of the sensor tags.

Where appropriate, the above-mentioned configurations and developments can be combined with each other as desired, as far as this is reasonable. Further possible configurations, developments and implementations of the invention also include combinations, which are not explicitly mentioned, of features of the invention which have been described previously or are described in the following with reference to the embodiments. In particular, in this case, a person skilled in the art will also add individual aspects as improvements or supplements to the basic form of the present invention.

The present invention is described in greater detail in the following on the basis of the embodiments shown in the schematic figures of the drawings, in which:
- Fig. 1: shows schematically a possible exemplary embodiment of an induction driven sensor according to an aspect of the present invention;
- Fig. 2: shows a possible exemplary implementation of a low power consumption electrical sensor element within an induction driven sensor according to an aspect of the present invention;
- Fig.3: shows schematically a possible exemplary embodiment a test apparatus using induction driven sensors according to an aspect of the present invention;
- Fig. 4: shows a flow chart for illustrating a possible exemplary embodiment of a method for sensing substances according to an aspect of the present invention.

The appended drawings are intended to provide further understanding of the embodiments of the invention. They illustrate embodiments and, in conjunction with the description, help to explain principles and concepts of the invention. Other embodiments and many of the advantages mentioned become apparent in view of the drawings. The elements in the drawings are not necessarily shown to scale.

In the drawings, like, functionally equivalent and identically operating elements, features and components are provided with like reference signs in each case, unless stated otherwise.

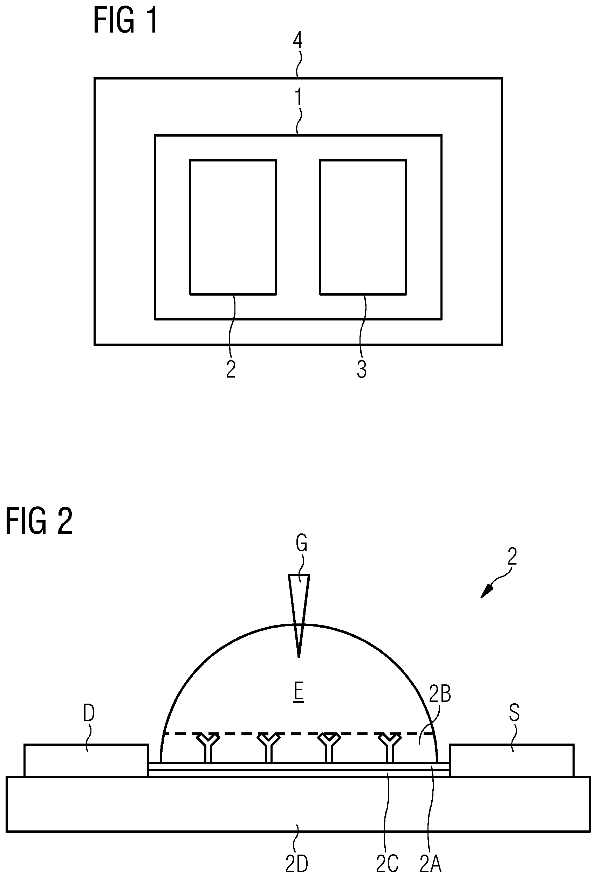

Fig. 1 schematically illustrates a possible and exemplary embodiment of an induction driven sensor 1 according to an aspect of the present invention.

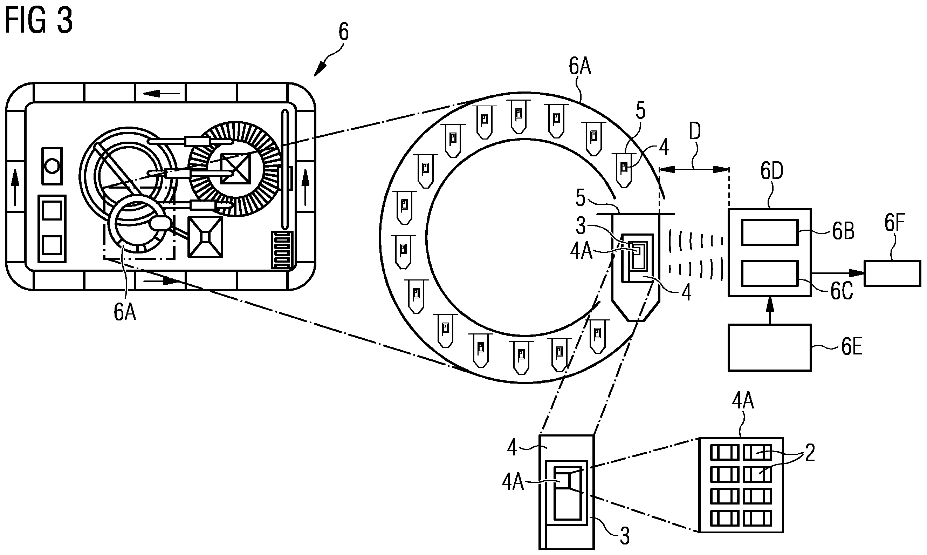

The induction driven sensor 1 comprises at least one low power consumption electrical sensor element 2 adapted to sense substances within a sample to be analysed provided. The sample to be analysed is contained in a sample container 5 which can be placed in a container holder of a test apparatus 6 as illustrated in Fig.3.

The sample can comprise a volume of any kind of liquid to be analysed for substances within this liquid or fluid. The fluid can be a body fluid of a person such as blood or e.g. a liquid taken from the environment or from an industrial process.

The induction driven sensor 1 further comprises an induction driven power supply structure 3 adapted to remotely power and read out the low power supply electrical sensor element 1. A multiplexed sensor element array 4A comprising a predetermined number of sensor elements 2 can be formed on a sensor tag 4 as also illustrated in Fig.3. The sensor tag 4 can be attached (e.g. glued) on the inside of the sample container 5.

In a possible embodiment of the induction driven sensor 1 the induction driven power supply structure 3 is patterned alongside the at least one power consumption electrical sensor element 2 on a flexible sensor tag 4. The flexible sensor tag 4 is provided within a sample container 5.

In a possible embodiment of the induction driven sensor 1 the induction driven power supply structure 3 comprises an RFID structure. Other near field communication technologies can be used in alternative implementations.

In a possible embodiment of the induction driven sensor 1 the low power consumption electrical sensor element 2 is adapted to receive for its operation an inductive power supply from the induction driven power supply structure 3 in response to external excitation radio waves. The induction driven power supply structure 3 comprises at least one integrated inductivity.

In a possible embodiment the amplitude and frequency of the external excitation radio wave is predetermined and fixed. In a preferred alternative embodiment the amplitude and/or frequency of the external excitation radio wave is adjustable.

The low power consumption electrical sensor element 2 is adapted to generate during its operation a measurement signal in response to electrically charged substances of the sample to be analysed. This measurement signal generated by the low power consumption electrical sensor element 2 during its operation can be detected by a radio wave detector 6C of a test apparatus 6 as changes in the excitation field as illustrated in Fig.3

In a preferred embodiment of the induction driven sensor 1 the low power consumption electrical sensor element 2 comprises at least one graphene based field effect transistor (GFET) as illustrated in Fig.2. The electrical power consumption of a GFET sensor element 2 is in a possible embodiment in the order of microwatts, i.e. less than 1 mWatt.

The induction driven sensor 1 comprises in a preferred embodiment a low power consumption electrical sensor element 2 formed by a flexible GFET. The electrical low power consumption sensor element 2 can be placed into a cuvette or other, sample container. Electric sensor usually need electrical connections to a power-supply and/or to read-out electronics. However, the low power requirements of GFET sensor elements 2 make it possible to remotely power and read out these devices via induction/RFID technology, thus greatly simplifying hardware requirements.

The GFET forming the low power consumption electrical sensor element 2 the induction driven sensor 1 can be fabricated on a Si/SiO2 substrate with metal contacts. The graphene itself can for instance be deposited onto the wafer through chemical vapour deposition (CVD). The first step to CVD is the decomposition of the carbon source at high temperatures.

Graphene is a substance composed of carbon atoms forming a crystal lattice with one atom in thickness. Graphene based FETs (GFET) are a subclass of FETs that have additional useful properties that make them suitable for applications with high cost pressures. The GFET sensor element 2 can be applied to a variety of carrier materials, including flexible plastics The GFET sensor element 2 can readily be modified chemically (e.g. with antibodies against antigens of interest) and is highly biocompatible.

The GFET forming the low power consumption electrical sensor element 2 of the induction driven sensor 1 comprises several key components including:
- a graphene layer 2A responsible for the transport of electrical current and the transduction of sensing events,
- a set of at least three electrodes D, S, G as required to operate a transistor,
- a delivery system allowing tested samples to reach the graphene layer 2A, and
- a layer 2B of recognition elements on the surface of the graphene layer 2A allowing for the specific capture of targeted substances.

The graphene based field effect transistor (GFET) sensor element 2 comprises the graphene layer 2A provided on a dielectric top layer 2C of a substrate 2D. A sensitive recognition layer 2B of the graphene based field effect transistor (GFET) sensor element 2 is adapted to capture at least one target substance in the sample to be analysed. This layer is provided on the graphene layer 2A of said graphene based field effect transistor (GFET) sensor element 2. The sensitive recognition layer 2B of the graphene based field effect transistor (GFET) sensor element 2 comprises in a possible embodiment receptors adapted to capture at least one target substance in the sample to be analysed.

Field effect transistors comprise two electrodes, i.e. a source electrode S and drain electrode D, which are connected by a semiconductor. By applying a voltage to a 3rd electrode (reference electrode), which is connected to the gate G via a conducting liquid or electrolyte E, a conduction channel is formed within the semiconductor and an electrical current I can flow between the source electrode S and the drain electrode D. As a GFET does possess an open gate structure, changes in electrical charge near the surface, e.g. the presence of ions or other charged molecules results in an alteration of the measured source to drain current I. The GFET sensor element 2 of the sensor 1 provides a high sensitivity and is small enough to perform spatial multiplexing by patterning multiple GFET sensor elements 2 in close proximity to each other.

The graphene field effect transistor sensor element 2 of the sensor 1 uses a nearby electric field and its associated voltage differential to modulate the current flow. GFETs are generally devices with three terminals, or electrodes. A semiconductor channel runs between two of these electrodes - the source electrode S and the drain electrode D- while a third, called the gate electrode G, acts as the control. The voltage differential applied to the gate electrode G allows or blocks the charge transport through the semiconductor channel depending on its direction and strength. In a possible embodiment the gate electrode G comprises an external gate electrode. In an alternative embodiment the gate electrode G can also be integrated on chip.

The graphene field-effect transistor sensor element 2 of the sensor 1 comprise a graphene channel (e.g. tens of microns in size) or a graphene layer 2A between the source electrode S and the drain electrode D. Being graphene, i.e. a lattice of carbon atoms that can be only one atom thick, the channel or layer in the GFET sensor element 2 has a high sensitivity, which can be exploited for sensing substances in a sample to be analyzed.

While the carbon in the graphene layer or graphene channel 2A of the GFET sensor element 2 typically does not readily react or bind with most materials, surface modification protocols have been developed to add receptors such as amino acids, antibodies or enzymes. Molecules can then be attached to these sites through covalent bonding, electrostatic forces or Van der Waals forces.

The two-dimensional structure of graphene of the GFET sensor element 2 of the induction driven sensor 1 has a number of benefits over conventional bulk semiconductors, such as silicon, used in standard FETs. Because most conventional semiconductor transistor sensors are three-dimensional, electric charge changes at the surface of the channel do not always penetrate deeper into the device. This can dramatically limit the response sensitivity of the device. On the other hand, as the graphene layer or graphene channel 2A in the GFET sensor element 2 is in a preferred embodiment only one carbon atom thick, i.e. the entire channel or layer form the surface, which directly exposes the graphene channel or graphene layer 2A to any molecules present in the nearby environment.

In order to form the GFET to the low power consumption electrical sensor element 2 of the induction driven sensor 1 graphene can be transferred on a planar substrate that provides physical support to the thin nanomaterial as well as to the electrodes. The substrate 2D, or at least its top layer 2C, is normally made of a dielectric or other insulating material to avoid unwanted electrical connections between the different electrodes placed on its surface.

The design of the GFET sensor element 2 includes at least three electrodes, in order to operate as a field-effect transistor. The first two electrodes, i.e. the source electrode (S) and the drain electrode (D), make direct contact with the graphene and enable the flow of electrical current I in the graphene through the application of a difference of electrical potential between them.

The source electrode S and drain electrode D of the GFET sensor element 2 are made of conductive material, typically a metal. The third electrode of the GFET, i.e. the gate electrode G, is placed in close proximity to the graphene layer 2A but not in direct contact. A potential difference is applied between the gate electrode G and the drain electrode D (or the source electrode D) of the GFET sensor element 2 to modulate the density and polarity of charge carriers in the graphene layer 2A.

The GFET forming the low power consumption electrical sensor element 2 of the induction driven sensor 1 can be used as a sensor element because the electrical conductance of the graphene layer or graphene channel 2A is sensitive to electrostatic changes in its environment, however the affinity between the graphene layer and other molecules is not specific.

To engineer the specificity of the GFET sensor element 2 of the induction driven sensor 1, the surface of the graphene layer 2A is functionalized in a possible embodiment with molecules of a recognition layer 2B able to specifically recognize and capture a substance of a target analyte. These recognition molecules can be referred to as probe molecules. The coverage of the graphene layer or graphene surface 2A with probe molecules is in some embodiments incomplete, in which case passivation strategies can be used to block nonspecific interactions with graphene.

For protein detection, antibodies can be used as probes, due to their high specificity and affinity for their antigen. For instance, if the GFET sensor element 2 is functionalized with antibodies it can been used to detect proteins identified as cancer biomarkers. Nucleic acid targets can be detected via hybridization with their complementary sequence.

The GFET-based sensor element 2 of the induction driven sensor 1 relies constitutively on electrical measurements, specifically measurements of the electrical current in the device channel - i.e. the graphene layer 2A. The general working principle of GFET sensor element 2 is that the density of charge carriers in the channel (and hence the electrical current) is modulated by the local electrostatic field, which is itself altered by physical or chemical changes in the environment around the channel.

In all cases, the detection principle of GFET sensor element 2 of the induction driven sensor 1 is based on a change in electrical metrics induced by changes in the environment of the sensor element 2. This can be exploited to detect the capture of bio-molecular species at the surface of the sensor element 2. Graphene is a particularly good choice for forming sensor element 2 because its atomically thin geometry makes its electrical conductance remarkably responsive to environmental effects, such as the capture or accumulation of biological analytes near the surface.

In practice, the electrical current I through the GFET sensor element 2 of the induction driven sensor 1 is also controlled by voltages applied to the source, drain and gate electrodes. The potential applied between the source electrode S and the drain electrode D generates the flow of charge carriers along the channel, while the gate voltage modulates the electric field across the channel - and thus the charge carrier density contributing to the electrical current I.

The GFET sensor element 2 of the induction driven sensor 1 comprises three standard operation curves including transfer curves (current vs. gate bias), output curves (current vs. drain-source bias) and time series (current vs. time) with fixed drain and gate voltages. In sensing applications, the effect of the analyte on such electrical curves can be monitored either a posteriori, by comparing a given metric before and after exposure to the sample, or in real-time by recording dynamic time series of the electrical current I.

In a possible embodiment an RFID structure 3 is patterned alongside the GFET sensor element 2 of the induction driven sensor 1, allowing for operation and read-out of the sensor 1 without direct electrical contact. The GFET sensor element 2 is operated via an external excitation source (radio waves) while the generated measurement signal is detected as changes in the excitation field.

The Radio Frequency Identification (RFID) enables fast and automatic data acquisition using electromagnetic waves with the help of transmitter and receiver units.

The invention further provides according to a further aspect a test apparatus 6 as illustrated in Fig.3.

The test apparatus 6 comprises a holder 6A adapted to hold multiple sample containers 5 each adapted to receive samples to be analysed by said test apparatus 6. Each sample container 5 comprises at least one sensor tag 4 having an induction driven sensor 1 including at least one low power consumption electrical sensor element 2 adapted to sense substances within a sample to be analysed within a sample container 5 and including an induction driven power supply structure 3 adapted to remotely power and read out the low power supply electrical sensor element 2 of the induction driven sensor 1.

The test apparatus 6 illustrated in the embodiment of Fig. 3 comprises a rotation unit 6A holding multiple sample containers 5 each adapted to receive samples to be analysed by said test apparatus 6. Each sample container 5 comprises at least one sensor tag 4 having an induction driven sensor 1 according to the first aspect of the present invention consisting of at least one GFET sensor element 2 and an associated induction driven power supply structure 3, in particular an RFID structure.

In a possible embodiment of the induction driven sensor 1 the sample to be analysed is in physical contact with the low power consumption electrical sensor element 2 of the induction driven sensor 1 of the sensor tag 4. The sample to be analysed is contained in a sample container 5, in particular in a cuvette. The cuvette (French: cuvette, lit. 'little vessel') is a small tube-like container with straight sides and a circular or square cross-section. The cuvette can be sealed at one end, and made of a clear, transparent material such as plastic, glass, or fused quartz.

The low power consumption electrical sensor element 2 of the induction driven sensor 1 can be provided in the interior of the sample container 5. The sensor tag can be attached to the inner wall of the sample container 5 so that the low power consumption electrical sensor element 2 comes into physical contact with the sample placed into the sample container. The sample to be analysed comprises typically a liquid filled into the sample container 5. Each sample container 5 may comprise one or more attached sensor tags 4. The sensor tag 4 can comprise a multiplexed GFET structure with a predetermined number of GFET sensor elements 2. This approach enables a multiplexed, high-throughput electrical sensor 1 that meets the cost-requirements for central lab applications.

The multiplexed GFET structure allows to read a number of biomarkers from the same sample to increase the throughput of the test apparatus 6. There are two main possibilities to read multiple biomarkers from a single sample. In a high-throughput system a sample can split into multiple sample containers 5, each having a sensor tag 4 measuring a biomarker. Alternatively, a multi well plate reader based systems can be used (spatial multiplexing).

The GFET sensor element 2 is capable to measure an electrical charge near a surface, thereby removing the need for secondary labelled antibodies or similar molecules as reporters.

GFET exhibits ambipolar charge transport behaviour in which electrons and holes can be modulated using an external electric field. Its two-dimensional structure consisting of a graphene monolayer being one atom thick facilitates strong responses to charged molecules present on the surface. More importantly, most biomolecules are naturally charged and so GFET sensors elements 2 can allow intrinsically label-free detection.

The readout signal of a GFET sensor element 2 is electrical and therefore compatible with electrical measurements based on standard electronic circuits.

Spatial multiplexing of GFET sensor elements 2 can be used to detect multiple analytes within a single sample container 5 or sample cuvette. This increases the throughput of the test apparatus 6.

In a possible embodiment of the test apparatus 6 at least one sample container rotation unit 6A is adapted to rotate a set of sample containers 5 such that the sample containers 5 pass sequentially at a distance D a radio wave excitation source 6B of the test apparatus 6 adapted to apply excitation radio waves to the sensor tags 4 attached to the sample containers 5.

In a possible embodiment of the test apparatus 1 a radio wave detector 6C of the test apparatus 6 is adapted to detect changes in the excitation field in response to a measurement signal generated by the low power consumption electrical sensor element 2 of the induction driven sensor 1 of the sensor tag 4 attached to the sample container 5 and being close to the radio wave detector 6C of the test apparatus 6.

The radio wave excitation source 6B and the radio wave detector 6C can form part of a reader 6D being controlled by a controller 6E of the test apparatus 6.

The detected measurement signal can be converted into measurement data processed by a processor 6F of the test apparatus 6 to determine a type and/or concentration of substances within the samples. The measurement signal and/or the determined type and concentration of substances can be output via a data interface to a cloud and/or via a display of the user interface of the test apparatus 6 to a user.

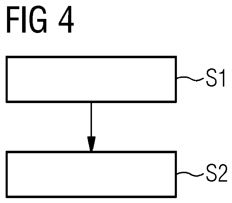

The invention provides according to a further aspect a method for sensing substances within a sample to be analysed as illustrated in the flow chart of Fig. 4.

In the illustrated embodiment of Fig. 4 the method comprises two main steps S1, S2.

In a first step S1 the sample to be analysed is supplied or delivered by bringing it into physical contact with a low power consumption electrical sensor element 2 of an induction driven sensor 1 adapted to sense substances within the sample.

The sample to be analysed can be delivered by filling it into a sample container 5 such as a cuvette. This can involve additional sample preparation steps such as dilution, washing or incubation.

The filling or refilling of the sample container 5 with a sample to be analysed from a sample source can be controlled by a controller 6E of the test apparatus 6.

The sample container 5 is held by a sample container holder 6A of the test apparatus 6 as illustrated in Fig.3 and can be moved into the vicinity of a reader 6D of the test apparatus 6, in particular an RFID reader.

In a second step S2 of the sensing method an excitation radio wave is applied to an induction driven power supply structure 3 of the induction driven sensor 1 of a sensor tag 4 attached to the sample container 5 to remotely power and read out the low power supply electrical sensor element 2 of the induction driven sensor 1.

The sensor element 2 of the induction driven sensor 1 is operated via an external excitation source (i.e. a transmitter of the reader providing radio waves) while the measurement signal generated by the sensor element 2 is detected as changes in the excitation field by a receiver of the reader 6D. Electrical energy induced by the excitation radio waves of the electromagnetic excitation field can be stored temporarily in a capacitor and used to supply the at least one low power supply electrical sensor element 2.

The transmitter and receiver of the reader 6D can be controlled by the controller 6E of the test apparatus 6. The controller 6E can also control the filling of the sample containers 5 with samples to be analysed. The filling can be performed in a possible implementation from one or more sample sources. The filled container 5 is rotated towards the reader 5D.

In a further possible embodiment the controller 6E is adapted to control the filling rate and the rotation speed of the rotating container holder 6A.

In a still further embodiment the controller 6E does also control the signal amplitude and/or the frequency of the excitation radio waves applied to the induction driven power supply structure 3 of the induction driven sensor 1. This provides more flexibility with regards to different implementations of sensor tags 4. Further an amplitude variation of the amplitude of the excitation radio wave can be used to compensate for variable distances between the reader 6D and the sample containers 5 in its vicinity and/or to compensate for varying sensitivities of sensor elements 2 with respect to substances within the sample to be analysed. It can also be used to read out more than one sensor tag 4 concurrently to increase the throughput of the test apparatus 6.

In a still further embodiment the induction driven power supply structures 3 of different induction driven sensors 1 of sensor tags 4 attached to a sequence of sample containers 5 held by a rotating sample holder 6A of the test apparatus 6 are structured such that they operate at different excitation frequencies. This allows to read out several sensor tags 4 of rotating sample containers 5 simultaneously to increase the rotation speed and thereby also increasing the throughput of the test apparatus 6.

The detected measurement signal output by the reader 6D of the test apparatus 6 can be converted into measurement data for further processing.

The sensing or measurement approach presented above can be applied to any type of electrically powered sensor element 2 as long as the power requirement of the respective sensor element 2 are compatible with a near field induction technology, in particular with the RFID technology.

The invention provides according to a further aspect a sample container 5 comprising an attached sensor tag 4 with an induction driven sensor 1 having at least one low power consumption electrical sensor element 2. The sensor element 2 is adapted to sense substances within a sample to be analysed filled into the sample container 5 and having an induction driven power supply structure 3. The induction driven power supply structure 3 is adapted to remotely power and read out the low power supply electrical sensor element 2 of the induction driven sensor 1 of the sensor tag 4 attached to the sample container 5.

The invention provides according to a further aspect a sensor tag 4 with an induction driven sensor 1 having at least one low power consumption electrical sensor element 2. The low power consumption electrical sensor element 2 is adapted to sense substances within a sample to be analysed filled into a sample container 5 and having an induction driven power supply structure 3 adapted to remotely power and read out the low power supply electrical sensor element 2 of the induction driven sensor 1 of the sensor tag 4.

The invention further provides a computer program product comprising instructions stored on a data carrier of the computer program product and adapted to perform the sensing method according to the third aspect of the present invention when executed on a controller of the test apparatus 6.

The program can control the supply of samples within sample containers 5 to the low power consumption electrical sensor element 2 of the induction driven sensor 1 and the application of excitation radio waves to the low power supply structure 3 of said induction driven sensor 1.

Although the present invention has been described in the above by way of embodiments, it is not limited thereto, but rather can be modified in a wide range of ways. In particular, the invention can be changed or modified in various ways without deviating from the core of the invention.

Independent of the grammatical term usage, individuals with male, female or other gender identities are included within the term.

## Claims

1. An induction driven sensor (1), the induction driven sensor (1) comprising:
at least one low power consumption electrical sensor element (2) adapted to sense substances within a sample to be analysed that are filled into a sample container (5); and
an induction driven power supply structure (3) adapted to remotely power and read out the low power supply electrical sensor element (2).

2. The induction driven sensor according to claim 1, wherein the induction driven power supply structure (3) is patterned alongside the at least one low power consumption electrical sensor element (2) on a flexible sensor tag (4).

3. The induction driven sensor according to claim 2, wherein the flexible sensor tag (4) is attached to the sample container (5).

4. The induction driven sensor according to any of the preceding claims, wherein the induction driven power supply structure (3) comprises a near filed induction structure, in particular an RFID structure.

5. The induction driven sensor according to any of the preceding claims, wherein the low power consumption electrical sensor element (2) is adapted to receive for its operation an inductive power supply from the induction driven power supply structure (3) in response to external excitation radio waves.

6. The induction driven sensor according to any of the preceding claims, wherein the low power consumption electrical sensor element (2) is adapted to generate during its operation a measurement signal in response to electrically charged substances of the sample to be analysed.

7. The induction driven sensor according to claim 6, wherein the measurement signal generated by the low power consumption electrical sensor element (2) during its operation is detected by a radio wave detector as changes in the excitation field.

8. The induction driven sensor according to any of the preceding claims, wherein the low power consumption electrical sensor element (2) comprises a graphene based field effect transistor sensor element.

9. The induction driven sensor according to claim 8, wherein the graphene based field effect transistor sensor element (2) comprises a graphene layer (2A) provided on a dielectric top layer (2C) of a substrate (2D).

10. The induction driven sensor according to claim 9, wherein a sensitive recognition layer (2B) adapted to capture at least one target substance in the sample to be analysed is provided on the graphene layer (2A) of said graphene based field effect transistor sensor element (2).

11. The induction driven sensor according to claim 10, wherein the sensitive recognition layer comprises receptors adapted to capture at least one target substance in the sample to be analysed.

12. The induction driven sensor according to any of the preceding claims, wherein the sample to be analysed is brought into physical contact with the low power consumption electrical sensor element (2) by filling it into the sample container (5), in particular a cuvette.

13. A test apparatus (6), the test apparatus (6) comprising:
multiple sample containers (5) arranged and configured to receive samples to be analysed by said test apparatus (6), wherein each sample container (5) comprises a sensor tag (4) having an induction driven sensor (1) according to any of the claims 1 to 12.

14. The test apparatus according to claim 13, further comprising:
a sample container holding unit (6A) adapted to hold and rotate the sample containers (5) such that the sample containers (5) pass sequentially a radio wave excitation source (6B) of the test apparatus (6) adapted to apply excitation radio waves to the sensor tags (4) attached to the sample containers (5); and/or
a radio wave detector (6C) adapted to detect changes in the excitation field in response to a measurement signal generated by the low power consumption electrical sensor element (2) of the induction driven sensor (1) of the sensor tag (4) attached to the sample container (5) passing the radio wave detector (6C).

15. A method for sensing substances within a sample to be analysed filled within a sample container (5), in particular by employing an induction driven sensor (1) according to any of the claims 1 to 12, the method comprising:
delivering (S1) the sample to be analysed to a low power consumption electrical sensor element (2) of an induction driven sensor (1) adapted to sense substances within the sample; and
applying (S2) an excitation radio wave to an induction driven power supply structure (3) of said induction driven sensor (1) to remotely power and read out the low power supply electrical sensor element (2) of said induction driven sensor (1) .
